(19) Europäisches Patentamt
European Patent Office
Office européen des brevets

(11) Publication number: 0 216 553
A2

(12) **EUROPEAN PATENT APPLICATION**

(21) Application number: 86306911.8

(22) Date of filing: 08.09.86

(51) Int. Cl.[4]: C 07 D 219/04
C 07 D 401/12, G 01 N 33/58

(30) Priority: 06.09.85 US 773377

(43) Date of publication of application: 01.04.87 Bulletin 87/14

(84) Designated Contracting States: AT BE CH DE FR GB IT LI LU NL SE

(71) Applicant: INTEGRATED GENETICS, INC.
51 New York Avenue
Framingham Massachusetts(US)

(72) Inventor: Septak, Michael J.
50 E. Bluff Road
Ashland Massachusetts 01721(US)

(74) Representative: Sheard, Andrew Gregory et al,
Kilburn & Strode 30, John Street
London WC1N 2DD(GB)

(54) **Acridine ester and acridinium ester synthesis.**

(57) A method of synthesis of acridinium esters comprises first preparing an acridine ester from a reactive derivative of a 9-carboxyacridine and subsequently N-alkylating the acridine ester.

EP 0 216 553 A2

Croydon Printing Company Ltd

# ACRIDINE ESTER AND ACRIDINIUM ESTER SYNTHESIS

This invention relates to acridine ester and acridinium ester synthesis.

Acridinium esters are chemiluminescent compounds which can be used to label macromolecules, for example by their chemical linkage to proteins or modified nucleic acids (Campbell et al. European Patent Application No. 0082636 and Mock et al. European Application; entitled "Labelled Nucleic Acids", 86306305.3 filed 15th August 1986, assigned to the same assignee as the present application, herein incorporated by reference). Compared to the previously utilized radioactive labelling of such macromolecules such ester-labels allow the relatively safe detection of the macromolecules without significantly affecting their binding properties to other macromolecules.

The synthesis of acridinium esters has been described by Weeks et al. (1983, Clinical Chemistry, 29:1474) and is shown in Fig. 1. Referring to Fig. 1, generally acridine - 9 - carbonyl chloride (species 3) is synthesized from the corresponding acridine (species 1) by the method of Lehmstedt et al. (Berichte, 1928, 61:2044), and the desired ester (species 7) is obtained by methylating species 6, which is derived from species 3 by a two step process in which a protecting group is firstly added (species 4) and then removed (species 5).

This invention provides an improved method for synthesizing acridine esters and also acridinium esters which can be directly linked to proteins or modified nucleic acids. Generally, in the method of the invention, a compound of the formula:

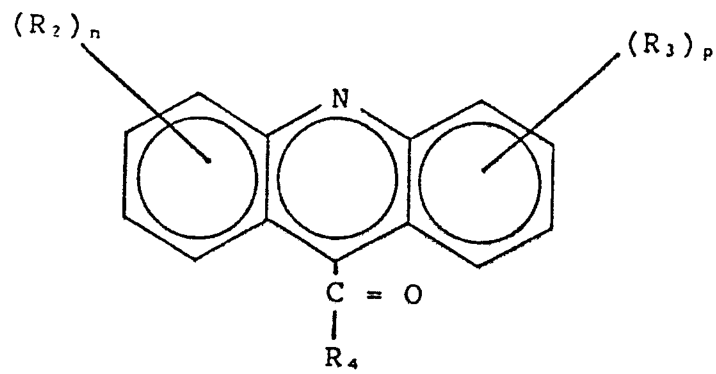

(1)

(where each $R_2$ and $R_3$, independently, is a substituent which does not interfere with the specific coupling chemistry of $R_4$ and the OH group of formula (2) below; each n and p independently, is 1 to 4, inclusive, provided that each $R_2$ and $R_3$ can be the same or different; and $R_4$ is a leaving group for example, n-hydroxysuccinimide, imidazole, or analog thereof (e.g. triazole or tetrazole or a halide) is reacted with a second species of formula:

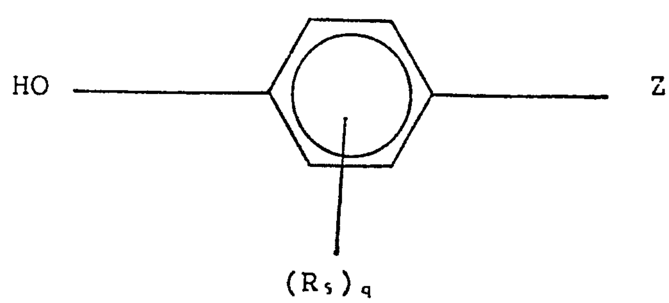

(2)

(where $R_5$ is any substituent which is substantially unreactive with the above species or with the rest of the molecule of which it is a part; for example $R_5$ is: ethyl, O-methyl, S-methyl, carboxymethyl, N,N-dimethylamide, halide, azide, NCS,

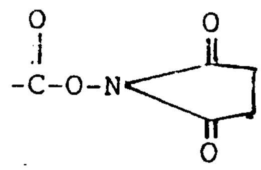

(3)

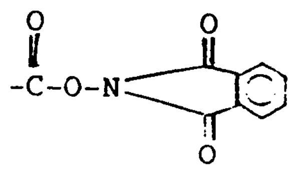

(4)

or

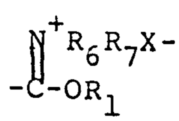

(5)

(where $R_1$ may be H, or an aliphatic chain of 1-30 (e.g. 1-6) carbon atoms inclusive, which may be substituted, e.g., by alkyl, alkenyl, alkynyl or aryl groups; X may be nothing, an anion such as a halide or a leaving group, for example: fluorosulphonate; and

where $R_6$ and $R_7$ are independently H or alkyl groups (of 1-30 (e.g. 1-6) carbon atoms), where $R_6$ and $R_7$ may be the same or different)); q is 1 to 4 inclusive; each $R_5$ can be the same or different; and Z is a group able to react with an amino group on a derivatized nucleic acid (Mock et al., id.) or protein, for example:

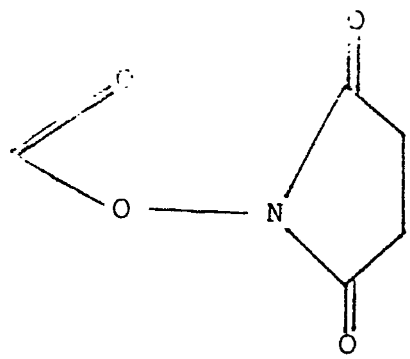

(6)

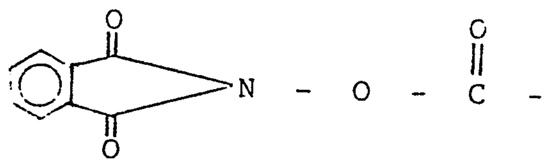

(7)

$N_3$,

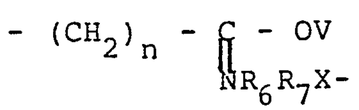

where V is an alkyl or phenyl group, $1 \leq n \leq 30$, and $R_6$, $R_7$ and X as are described above (8)

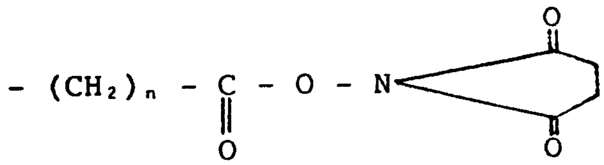 where $1 \leq n \leq 30$,

(9)

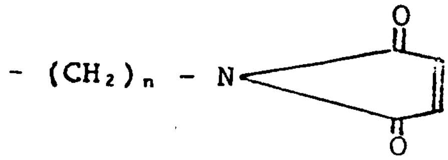 where $1 \leq n \leq 30$,

(10)

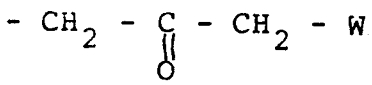 where W is a halogen, (preferably bromine) (11)

thiocyanate, or imidazole (or analogs thereof, such as triazole and tetrazole). The reaction may be carried out in a common solvent, for example anhydrous pyridine. The resulting species has the formula:

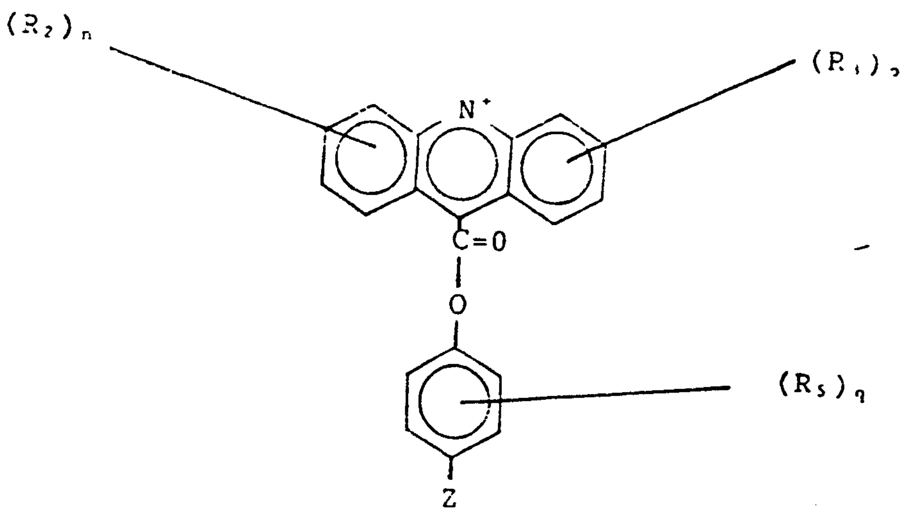

(12)

and may be purified e.g. according to polar separation (using flash chromatography) or hydrophobicity (using reverse phase chromatography). The pure species can then be alkylated, e.g. $C_1$-$C_6$ alkylated preferably with methylfluorosulphonate, methyliodide, or dimethyl sulphate, to produce the desired acridinium ester.

The above synthesis can be achieved without the need for protecting and deprotecting steps, can use commercially available starting materials, and has an overall yield of around 25%.

Other features and advantages of the invention will be apparent from the following description of the preferred embodiments therefrom, and from the claims.

## Description of the Preferred Embodiments

The drawings will first briefly be described.

### Drawings

Figure 1 is a flow diagram of the reaction scheme for the synthesis of acridinium esters as described by Weeks et al. (*id*).

Figure 2 is a flow diagram of a general reaction scheme of this invention, for the synthesis of acridine and acridinium esters.

### Structure

#### Acridinium Esters

Acridinium esters, including those useful for labelling modified nucleic acids, are described in Weeks et al. (*id.*), Campbell et al. European Patent 0082636, McCapra et al. British Patent 1,461,877 and Sheehan U.S. Patent 3,539,574, all hereby incorporated by reference. Acridinium esters useful in the invention have the general

formula shown in Fig. 2 as species IV. The relevant R, Z, and X groups are as described below.

Species I

Referring to Fig. 2, species I is generally substituted by non-nucleophilic species which will not interfere with the specific coupling of species I and II, to give species III; as described by Weeks et al. (id.). Specifically, $R_2$ and $R_3$ may be H, alkyl (an aliphatic chain of 1-30 (e.g. 1-6) carbon atoms), disubstituted amino (e.g., diethylamino, or any other alkyl substitution of 1-30 (e.g. 1-6) carbon atoms, where each alkyl group may be the same or different), alkoxyl, nitro, or halide; n and p are independently 1 to 4 and each $R_2$ and $R_3$ can be the same or different. $R_4$ is a leaving group, for example, N-hydroxysuccinimide, an imidazole, or a halide.

Species II

Referring to Fig. 2, species II is generally able to react with species I and to attach to modified nucleic acids or proteins. Specifically, $R_5$ is any substituent which is substantially unreactive with species I (so that the reaction between species I and II is directed at $R_4$ of species I and OH of species II), or with the rest of species II (so that species II dimers are not formed); for example $R_5$ is: ethyl, O-methyl, S-methyl, carboxymethyl, N,N-dimethylamide, halide, azide, NCS,

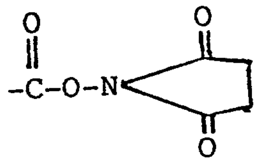

(3)

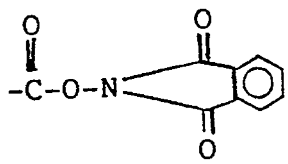

(4)

or

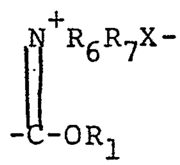

(5)

(where $R_1$ may be H, or an aliphatic chain, of 1-30 (e.g. 1-6) carbon atoms inclusive, which may be substituted by alkyl, alkenyl, alkynyl or aryl groups; X is nothing, a halide or a leaving group, for example, methyl fluorosulphonate; and where $R_6$ and $R_7$ are independently H or alkyl groups (of 1-30 carbon atoms), where $R_6$ and $R_7$ may be the same or different); q is 1 to 4 and each $R_5$ is the same or different. Z is a group able to attach to modified nucleic acids (Mock et al., id.) or protein, for example:

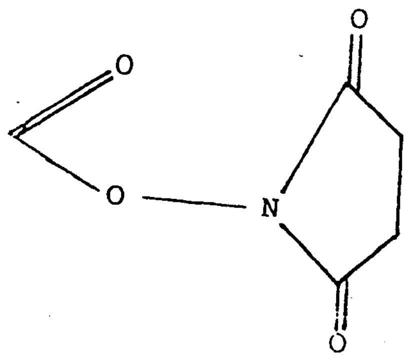

(6)

NCS,

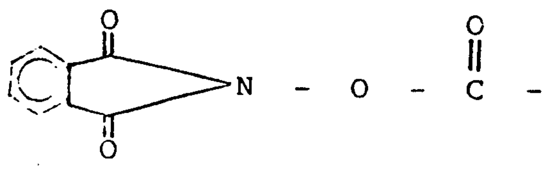

(7)

$N_3$,

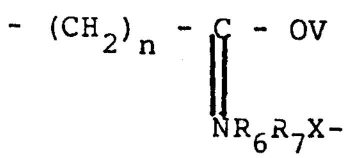

where V is an alkyl (preferably with 1 to 4 carbon atoms) or phenyl group, $1 \leq n \leq 30$, and $R_6$, $R_7$ and X are as described above, (8)

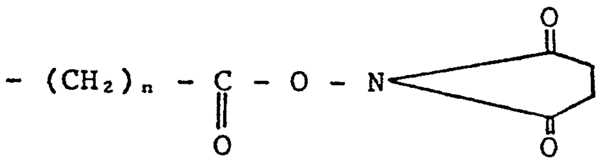 where $1 \leq n \leq 30$, (9)

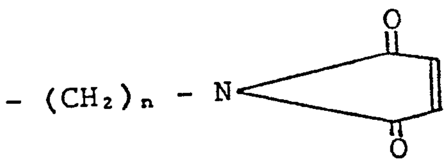 where $1 \leq n \leq 30$, (10)

$-CH_2-C(=O)-CH_2-W$ where W is a halogen, preferably bromine, (11) thiocyanate, or imidazole (or analogs thereof, such as triazole and tetrazole).

The phenyl group may be attached to Z via any spacer molecule which will not interfere with the specific reaction of species I and II. Such spacer preferably does not contain reactive species, such as hydroxyl, amino, carboxyl or thiol groups, which participates in undesired undirected reactions.

Alkylating Agents

Species III is alkylated by a alkylating agent to introduce the group $R_1X^-$, where $R_1$ and X are as described above. Preferred alkylating agents are methyfluorosulfonate, methyliodide or dimethylsulfate.

Synthesis

Referring to Fig. 2, species I is readily obtained from the corresponding acridine or acid, as shown in Fig. 1 (Lehmstedt et al., id.), and species II is readily available (Aldrich). The two species are mixed in the presence of anhydrous pyridine for approximately 15-60 minutes and the resulting species purified. This resulting species, species III, is generally purified from unreacted species I and II by polar separation or by hydrophobicity. Preferred is silica gel or neutral alumina flash chromatography or reverse phase C-18 chromatography. The final alkylation reaction is performed according to Weeks et al. (id.). An example of such a reaction is given below:

EXAMPLE

Acridine-9-carboxylic acid (870 mg) is added to thionyl chloride (10 ml) and the mixture refluxed in an oil bath for three hours. The reaction mixture is evaporated *in vacuo* on a rotary evaporator and the dry residue redissolved in anhydrous pyridine (50 ml), with heating, below 60°C. This solution is cooled to approximately 20°C in a water bath. p-hydroxy phenylpropionic acid n-hydroxy succinimide ester (1 g) is dissolved in anhydrous pyridine (10 ml) and added

to the acridine-9 carboxylic chloride-pyridine solution above, with stirring. The reaction mixture is stirred at 15-30°C for approximately 30 minutes and then poured into ice water (250 ml), whilst stirring. This mixture is transferred into a separating funnel and extracted with ethyl acetate (250 ml). The organic phase is separated, dried over anhydrous sodium sulphate, filtered and evaporated in vacuo. The residue is evaporated from anhydrous toluene (50 ml) three times and redissolved in anhydrous dimethoxy ethane (15 ml). The resulting solution is placed at 15-30°C for approximately 15 hours in a light-proof container. Any precipitate formed during this incubation is filtered away and the solution applied to a silica gel column (300 grams) and purified by flash chromography (W.C. Still et al., Journal of Organic Chemistry (1978), Vol. 43, 2923) with a solution of ethyl acetate and hexane (2:1) as an eluting solvent. Pure fractions are combined and filtered through a Teflon 47 mm 0.45 μm filter and evaporated in vacuo. The yield is approximately 36%. This is redissolved in anhydrous chloroform (25 ml) and methyl fluorosulphonate (1.1 ml) added to the mixture which is stirred at 15-30°C for approximately 20 hours. The precipitate formed is filtered off and washed three times with anhydrous toluene and two times with anhydrous dimethoxy ethane. The dried crystals are stored desiccated and protected from the light at -20°C. The final overall yield is approximately 556 mg. (25%) of pure acridine ester.

Other embodiments are within the following claims.

0216553

CLAIMS

1. A process for the preparation of an acridine ester of general formula III ⁄

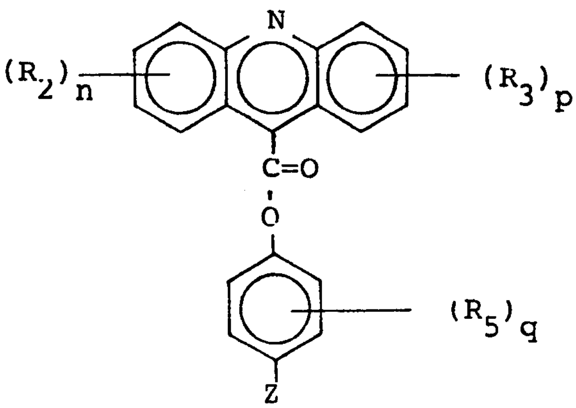

III

wherein $R_2$, $R_3$, n and p are as defined below for general formula I;

$R_5$ and q are as defined below for general formula II; and

Z represents a group able to react with an amino group of a derivatised nucleic acid or protein;

the process being characterised by reacting a compound of general formula I

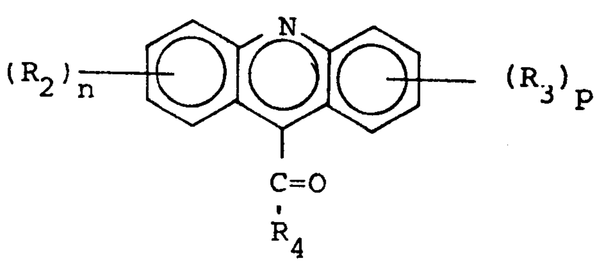

I

wherein each of $R_2$ and $R_3$ independently represent one or more substituents which may be the same as or different from each other and which will not interfere with the desired specific coupling chemistry of the compound of general formula I;

each of n and p is independently an integer of from 1 to 4; and

$R_4$ represents a leaving group;

with a compound of general formula II

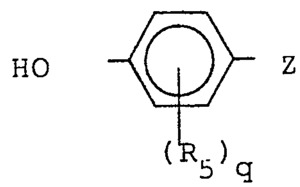

II

wherein $R_5$ represents one or more substituents which may be the same as or different from each other and which are unreactive with compounds of general formula I and unreactive with compounds of general formula II;

q is an integer of from 1 to 4; and

Z is as defined for general formula III.

2. A process for the preparation of an acridinium ester of general formula IV

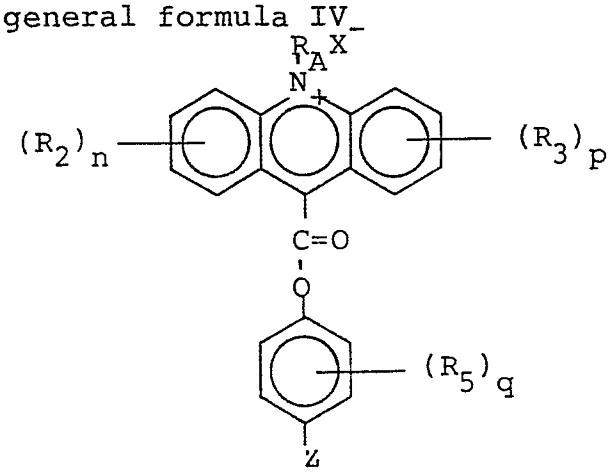

IV

wherein $R_2$, $R_3$, n and p are as defined below for general formula I;

$R_5$ and q are as defined below for general formula II;

Z represents a group able to react with an amino group of a derivatized nucleic acid or protein;

$R_A$ represents a hydrogen atom or a $C_1$-$C_6$ alkyl radical; and

$X^-$ represents an anion or a monovalent portion of an anion;

the process being characterised by reacting a compound of general formula I

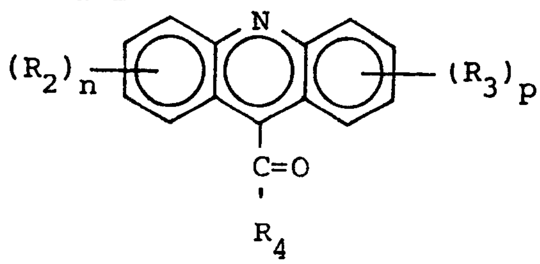

I

wherein each of $R_2$ and $R_3$ independently represent one or more substituents which may be the same as or different from each other and which will not interfere with the desired specific coupling chemistry of the compound of general formula I;

each of n and p is independently an integer of from 1 to 4; and

$R_4$ represents a leaving group;

with a compound of general formula II

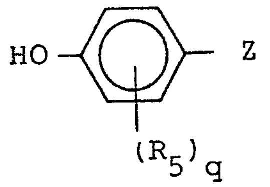

II

wherein $R_5$ represents one or more substituents which may be the same as or different from each other and which are unreactive with compounds of general formula I and unreactive with compounds of general formula II;

q is an integer of from 1 to 4; and

Z is as defined for general formula IV, to form an acridine ester of general formula III

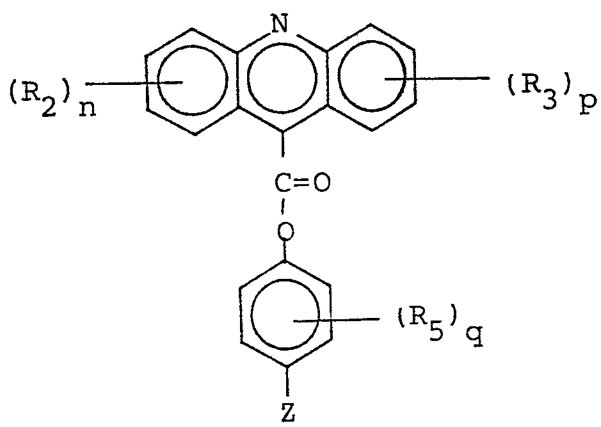

III

wherein $R_2$, $R_3$, n and p are as defined for general formula I

$R_5$ and q are as defined for general formula II; and

Z is as defined for general formula I

and subsequently either forming an acid addition salt of the compound of general formula III by reaction with an appropriate acid to form a compound of general formula IV wherein $R_A$ represents a hydrogen atom or N-alkylating the compound of general formula III with an N-alkylating agent containing the radical $R_A$, wherein $R_A$ represents a $C_1$-$C_6$ alkyl radical to form a compound of general formula IV wherein $R_A$ represents a $C_1$-$C_6$ alkyl radical.

3. A process as claimed in Claim 1 or 2, wherein the acridine ester is purified.

4. A process as claimed in Claim 3 wherein, the purifying comprises extraction with ethyl acetate.

5. A process as claimed in Claim 3 or 4 wherein the purifying further comprises flash chromatography or reverse phase chromatography.

6. A process as claimed in any one of Claims 1 to 5 wherein the compound of general formula I and the compound of general formula II are mixed for a period of 10 to 60 minutes at 15 to 30°C.

7. A process as claimed in Claim 2, wherein the compound of general formula III and the alkylating reagent are mixed for a period of 15 to 25 hours at 15 to 30°C.

8. A process as claimed in Claim 2 or 7, wherein the alkylating reagent is methyl fluorosulphonate, methyliodide, or dimethyl sulphate.

9. A process as claimed in any one of Claims 1 to 8, wherein $R_5$ represents: ethyl, O-methyl, S-methyl, carboxymethyl, N,N-dimethylamide, halide, azide, NCS,

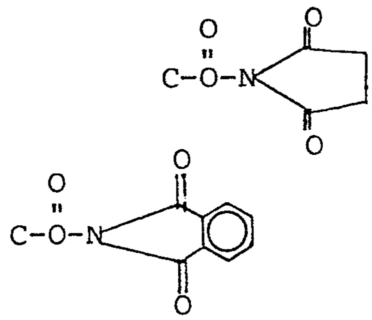

or

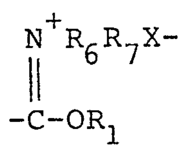

where $R_1$ represents a hydrogen atom or an aliphatic chain containing from 1 to 30 carbon atoms, inclusive, which may be substituted with one or more alkyl, alkenyl, alkynyl or aryl groups; X represents nothing, an anion such as a halide, or one of the leaving groups: perchlorate, fluorosulphonate, iodide and sulphate and each of $R_6$ and $R_7$ independently represent a hydrogen atom or an alkyl group of from 1 to 30 carbon atoms, where $R_6$ and $R_7$ may be the same or different.

10. A process as claimed in any one of Claims 1 to 9, wherein the substituent represented by Z includes one of the following amino-reactive functionalities:

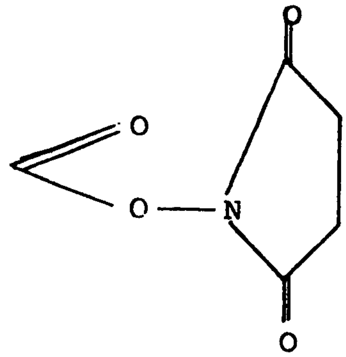 ;

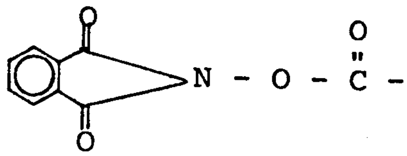 ;

$N_3$;

$-(CH_2)_n-C-OV$
$\qquad\qquad \|$
$\qquad\qquad N^+R_6R_7X^-$

where V represents an alkyl or phenyl group,

$1 \leq n \leq 30$, each of $R_6$ and $R_7$ independently represent a hydrogen atom or an alkyl group of from 1 to 30 carbon atoms, wherein $R_6$ and $R_7$ may be the same or different, and X is nothing, an anion such as halide or a leaving group.

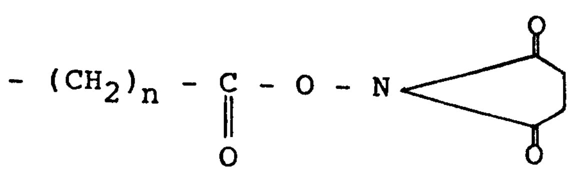 where $1 \leq n \leq 30$;

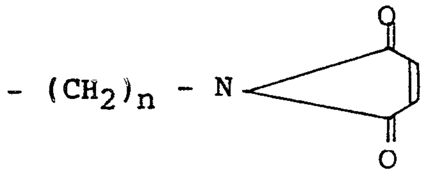

where $1 \leq n \leq 30$;

or

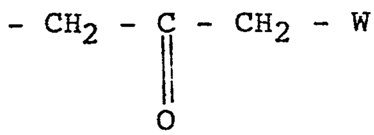

where W represents a halogen.

11. A process as claimed in any one of Claims 1 to 10 wherein the ester formation reaction is carried out in anhydrous pyridine.

12. A process as claimed in any one of Claims 1 to 11, wherein $R_4$ represents N-hydroxysuccinimide, imidazole or analogue thereof (e.g. tetrazole), or a halide.

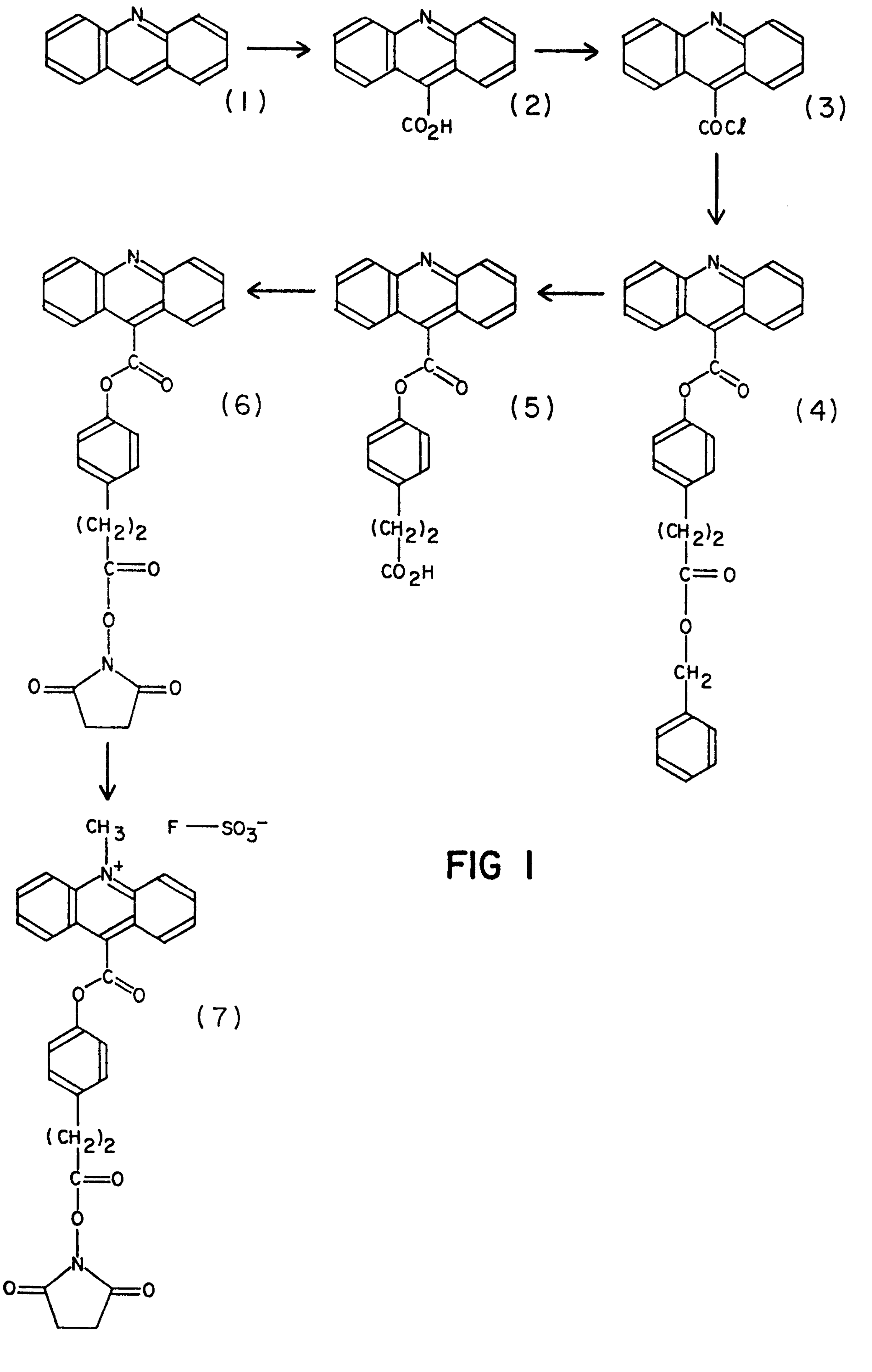

(1)

(2)

(3)

(4)

(5)

(6)

(7)

FIG 1

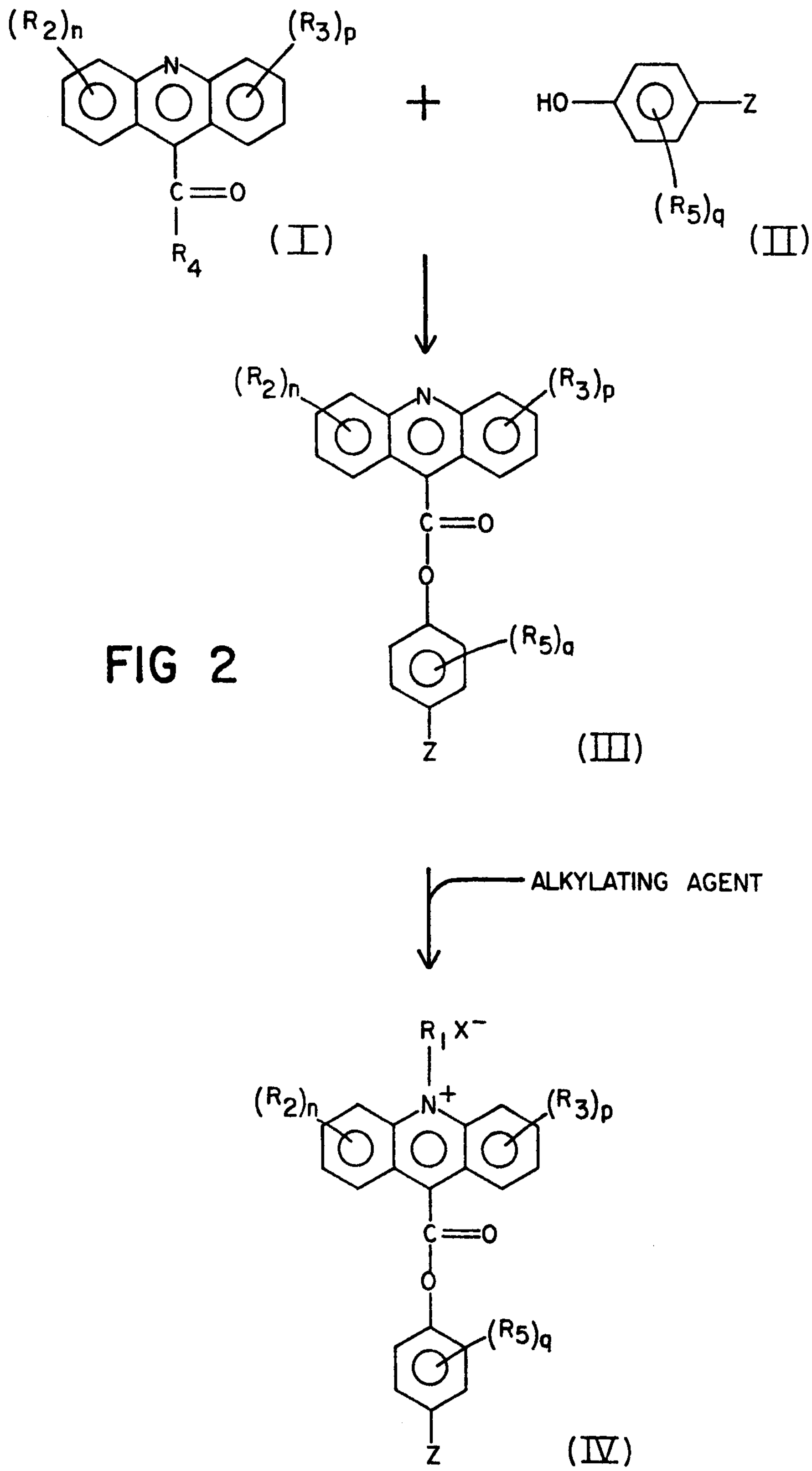


(I) (II) (III) (IV)

FIG 2